(19) Europäisches Patentamt
European Patent Office
Office européen des brevets

(11) Numéro de publication: **0 010 460**
**B1**

# (12) FASCICULE DE BREVET EUROPEEN

(45) Date de publication du fascicule de brevet: **27.01.82**

(51) Int. Cl.³: **C 07 D 207/08**, C 07 D 207/26, A 61 K 31/40

(21) Numéro de dépôt: **79400608.0**

(22) Date de dépôt: **04.09.79**

(54) **Nouveaux dérivés de pyrrolidine-2-méthanol, procédé pour leur préparation et médicaments les contenant.**

(30) Priorité: **22.09.78 FR 7827178**

(43) Date de publication de la demande: **30.04.80 Bulletin 80/9**

(45) Mention de la délivrance du brevet: **27.01.82 Bulletin 82/4**

(84) Etats contractants désignés: **BE CH DE FR GB IT LU NL SE**

(56) Documents cités:
**CHEMICAL ABSTRACTS, vol 67, no 18, 30-10-1967, page 8527, abrégé 90642w.
Columbus, Ohio, USA
A. M. LIKHOSHERSTOV et al. »Aza cycloalkanes. III. Tertiary α-azacycloalkylcarbinols. Synthesis and pharmacological activity«.**

(73) Titulaire: **PHARMINDUSTRIE, 35 Quai du Moulin de Cage, F-92231 Gennevilliers (FR)**

(72) Inventeur: **Legrand, Jean-Jacques, 59 Boulevard Saint-Marcel, F-75013 Paris (FR)**
Inventeur: **Renault, Christian, 17, Allée Réaumur, F-95150 Taverny (FR)**

(74) Mandataire: **Houssin, Jean et al, PRODUITS CHIMIQUES UGINE KUHLMANN Service Propriété Industrielle Tour Manhattan Cedex 21, F-92087 Paris La Defense 2 (FR)**

EP 0 010 460 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouveaux dérivés de pyrrolidine-2 méthanol, procédé pour leur préparation et médicaments les contenant

La présente invention concerne de nouveaux dérivés de pyrrolidine-2 méthanol, utilisables notamment comme hypolipémiants et plus particulièrment comme hypotriglycéridémiants. Certains de ces dérivés peuvent également trouver des applications comme psychotropes, plus particulièrement comme psychostimulants.

Les composés selon l'invention répondent à la formule générale:

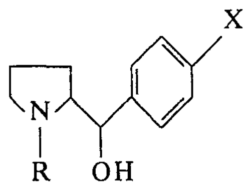

(I)

dans laquelle X représente un atome d'hydrogène, un atome d'halogène, un groupe alkoxy comportant de 1à 4 atomes de carbone, un groupe alkyle ou cycloalkyle comportant de 1à 8 atomes de carbone et R représente un atome d'hydrogène, un groupe alkyle comportant de 1 à 4 atomes de carbone, ou un groupe aralkyle (en particulier phénylalkyle) ou hydroxyalkyle dans lequel la chaîne alkyle comporte 1à 4 atomes de carbone.

Pour une signification déterminée des substituants R et X, il y a deux composés diastéréoisomères, un thréo et un érythro, répondant à la formule globale (I).

Les composés de formule (I) pour lesquels R est autre qu'un atome d'hydrogène peuvent être préparés par alkylation, sur l'atome d'azote, des composés correspondants de formule (I) pour lesquels R=H. Une telle réaction d'alkylation est effectuée au moyen d'un agent alkylant approprié, suivant des méthodes connues pour l'alkylation sur l'azote des amines secondaires, par exemple celles décrites dans WAGNER et ZOOK, Synthetic Organic Chemistry, p. 666 (Edition J. WILEY 1965).

Les produits de formule (I) pour lesquels R=H peuvent être préparés par réduction des produits de formule (II):

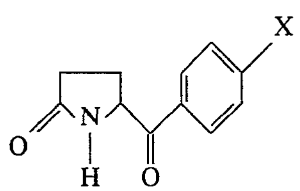

(II)

dans laquelle X a les mêmes significations que dans la formule (I).

On utilise comme agent réducteur des dérivés du bore tels que le diborane ou un hydrure métallique approprié tel que l'hydrure d'aluminium et de lithium, de préférence en excès. On opère au sein d'un solvant inerte tel que l'éther ou le tétrahydrofuranne, à une température située entre 20°C et la température d'ébullition du solvant utilisé. Une fois la réduction terminée, le mélange réactionnel est traité à l'eau, en présence d'hydroxyde de sodium et à basse température, le précipité minéral formé est séparé par filtration et le filtrat est évaporé.

Les procédés décrits ci-dessus conduisent généralement à un mélange des deux isomères thréo et érythro, isomères que l'on peut séparer par des méthodes classiques, physiques (cristallisation, chromatographie, etc.) ou chimiques (formation de sel et régénération de la base, etc.). L'isomère prépondérant est en général isolé facilement à partir du produit brut de la réaction par une simple recristallisation.

Une méthode avantageuse pour obtenir l'autre isomère consiste à isomériser l'isomère prèpondérant par action successive d'un chlorure d'acide, tel que le chlorure de thionyle, et de l'eau, selon des méthodes connues en soi, telles que celle décrite dans Journal of organic chemistry 33 1762 (1968).

Les composés de formule (I) sous forme de bases libres peuvent éventuellement être transformés en sels d'addition avec un acide minéral ou organique par action d'un tel acide au sein d'un solvant approprié.

Les composés de formule (II), qui sont des produits nouveaux, peuvent être préparés par réaction du chlorure de l'acide pyroglutamique (III) avec un dérivé benzénique de formule (IV), dans laquelle X a les mêmes significations que dans la formule (I), selon le schéma réactionnel:

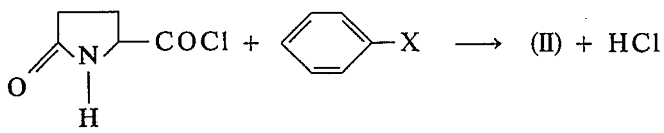


(III) (IV)

Cette réaction est effectuée en présence d'un catalyseur pris parmi les catalyseurs connus sous le nom de catalyseurs de Friedel et Crafts (halogénures métalliques, oxydes métalliques, iode, acides minéraux), dans des conditions telles que celles décrites par WAGNER et ZOOK, Synthetic Organic Chemistry, p. 317 (Edition J. Wiley — 1965). Une méthode avantageuse consiste à opérer avec le chlorure d'aluminium comme catalyseur, en l'absence ou en présence d'un solvant. Comme solvants utilisables on peut citer par exemple le tétrachlorure de carbone et le sulfure de carbone.

Le chlorure d'acide de formule (III) est un produit connu [cf. W. VOSS Z. Physiol, Chem. 204, 1 (1932)]. Il peut être préparé à partir de l'acide pyroglutamique par un procédé analogue à celui décrit dans J. Prakt. Chem. 5 (1957) p. 91—96.

Le rôle pathogène des lipoprotéines de très basse densité (en anglais very low density lipoproteins ou V.L.D.L.) dans la genèse et l'évolution des dislipémies (c'est-à-dire des troubles du métabolisme des lipides se traduisant par un taux anormalement élevé de lipides circulant dans le sang) justifie l'intérêt thérapeutique des produits susceptibles de freiner la production de ces lipoprotéines. Etant donné la richesse en triglycérides de ces lipoprotéines légères, il apparait qu'un des meilleurs moyens d'en réduire le taux plasmatique est de diminuer le taux des triglycérides circulants.

Les composés de formule (I) possèdent la propriété de réduire le taux des triglycérides circulants. En outre certains de ces composés appartenant à la série érythro présentent une action notable sur le système nerveux central, en particulier une action psychostimulante.

Les exemples suivants illustrent l'invention sans la limiter. Les résultats des analyses par résonance magnétique nucléaire (R.M.N.) figurant dans ces exemples ont été obtenus en mettant les produits en solution dans le deutérochloroforme CD $Cl_3$ et en utilisant comme référence le tétraméthylsilane.

## Exemple 1

### α-phényl pyrrolidine-2 méthanol (isomère érythro)

#### a) Préparation de la benzoyl-5 pyrrolidone-2

A une suspension de 142 g d'acide pyroglutamique dans 930 ml de chlorure d'acétyle on ajoute à −10°C, en 20 minutes, 300 g de pentachlorure de phosphore. On maintient la température à 0°C pendant 4 heures. On sépare le précipité rose formé et on le lave avec deux fois 100 ml de benzène.

Le chlorure d'acide ainsi obtenu (chlorure de l'acide pyroglutamique) est mis en suspension dans 3 l de benzène anhydre. One ajoute alors par petites fractions 300 g de chlorure d'aluminium. On chauffe 12 h. au reflux. On refroidit à température ambiante et on verse mélange réactionnel sur un mélange de 3 kg de glace et 300 ml d'acide chlorhydrique concentré. On agite 2 heures à température ambiante puis on sépare le précipité beige obtenu. On met ce dernier en suspension dans 150 ml d'acétate d'éthyle. On essore et on sèche sous vide. On obtient ainsi 121,5 g de benzoyl-5 pyrrolidone-2 fondant à 175°C.

#### b) Préparation de l'α-phényl pyrrolidine-2 méthanol (isomère érythro)

A une suspension de 26,4 g d'hydrure d'aluminium et de lithium dans 4 litres de tétrahydrofuranne anhydre on ajoute par petite fractions, sous atmosphère d'azote, 65,6 g de benzoyl-5 pyrrolidone-2 préparée comme indiqué ci-dessus. On porte alors au reflux sous agitation, pendant 2 heures. Puis on refroidit à 0°C et on ajoute lentement 30,5 ml d'eau puis 11 ml de solution 10N d'hydroxyde de sodium et enfin 110 ml d'eau. Après avoir agité 2 heures à température ambiante on sépare par filtration le précipité formé. Le filtrat obtenu est séché sur sulfate de magnésium et le solvant distillé sous pression réduite. On obtient 53 g d'α-phényl pyrrolidine-2 méthanol. Ces 53 g sont recristallisés dans 580 ml d'éther isopropylique. On obtient ainsi 36 g d'α-phényl-pyrrolidine-2 méthanol (érythro) fondant à 110°C.

Analyse élémentaire du produit obtenu:

| | | | |
|---|---|---|---|
| Calculé | C % 61,90 | H % 7,5 | N % 6,55 |
| Trouvé | C % 62,05 | H % 7,6 | N % 6,38 |

Exemple 2

Préparation de l'α-Phényl pyrrolidine-2 méthanol (isomère thréo)

A une solution de 52,4 g d'α-phényl pyrrolidine-2 méthanol (isomère érythro) dans 500 ml de chloroforme, refroidie à 0° C, in ajoute goutte-à-goutte 100 ml de chlorure de thionyle. On agite 1 heure à température ambiante. La solution est ensuite coulée sur 1,5 kg de glace et le mélange est agité 30 minutes. On décante. La phase aqueuse est alors chauffée au reflux pendant 2 heures puis refroidie à 0° C et amenée à pH = 11 par de l'hydroxyde de sodium. L'huile qui relargue est extraite par 4 fois 200 ml de chloroforme. On sèche la phase organique sur sulfate de magnésium. On évapore le solvant sous vide. Les 50,6 g d'huile obtenus sont dissous dans 300 ml d'éther éthylique anhydre. On ajoute goutte-à-goutte 60 ml d'une solution 5N d'acide chlorhydrique dans l'éther. On refroidit à 0° C et on agite 15 minutes. On sépare le précipité blanc obtenu. On le lave avec 3 fois 50 ml d'éther éthylique anhydre et on le sèche sous vide. On obtient ainsi 60 g de chlorhydrate d'α-phényl pyrrolidine-2 méthanol (thréo) brut fondant à 160° C.

Ces 60 g sont recristallisés dans 250 ml d'isopropanol. On obtient ainsi 52 g de chlorhydrate d'α-phényl pyrrolidine-2 méthanol (thréo) fondant à 176° C.

Analyse élémentaire du produit obtenu:

| | | | |
|---|---|---|---|
| Calculé | C % 61,90 | H % 7,50 | N % 6,55 |
| Trouvé | C % 61,81 | H % 7,62 | N % 6,39 |

Exemple 3

Préparation de l'α-Phényl (benzyl-1 pyrrolidine)-2 méthanol (isomère thréo)

On chauffe 1 h. au reflux une suspension de 11,7 g d'α-phényl pyrrolidine-2 méthanol (thréo), 11,3 g de bromure de benzyle et 10,8 g d'acétate de sodium dans 170 ml d'éthanol absolu. On refroidit ensuite à température ambiante, puis on chasse le solvant par distillation sous pression réduite. La masse pâteuse obtenue est reprise dans 100 ml d'eau. L'huile qui relargue est extraite par 2 fois 100 ml de chloroforme. Après avoir séché la phase organique sur sulfate de magnésium on élimine le solvant par distillation sous pression réduite. On obtient 17,6 g d'huile jaune que l'on dissout dans 100 ml d'éther de pétrole. Après une nuit à 0° C on sépare le précipité formé. On obtient ainsi 14,5 g d'α-phényl (benzyl-1 pyrrolidine)-2 méthanol (thréo) fondant à 190° C.

Spectre de R.M.N. du produit obtenu:

Proton en α de OH: déplacement chimique S = 4,4 ppm

$-CH_2-$ 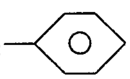 : $\delta$ = 3,7 et 3,4 ppm

Exemple 4

Préparation de l'α-phényl (méthyl-1 pyrrolidine)-2 méthanol (isomére thréo)

On chauffe, en autoclave, à 140° C un mélange de 8,9 g d'α-phényl pyrrolidine-2 méthanol (thréo), 1,7 ml d'acide formique à 99%, 5,4 g de formol à 30% en volume et 55 ml d'eau. Après une nuit à 140 C, on refroidit à température ambiante, on alcalinise le mélange réactionnel par addition d'une solution aqueuse à 38% d'hydroxyde de sodium et on ajoute à la solution aqueuse du carbonate de potassium jusqu'à saturation. L'huile qui relargue est extraite par 3 fois 100 ml de chloroforme. On sèche la phase organique sur sulfate de magnésium. On chasse le solvant par distillation sous pression réduite. On obtient 9,5 g d'une huile brun clair. L'huile obtenue est solubilisée dans 100 ml d'acétone et on ajoute 5,7 g d'acide fumarique dissous dans 30 ml d'acétone. On sépare par filtration le fumarate obtenu. On obtient ainsi 9,5 g de fumarate d'α-phényl (méthyl-1 pyrrolidine)-2 méthanol (thréo), qui fond à 156° C.

Spectre de R.M.N. du produit obtenu:

– Proton en α du OH: $\delta$ = 4,4 ppm
– $CH_3$: $\delta$ = 2,2 ppm

## Exemple 5

### Préparation de l'α-phényl (β-hydroxyéthyl-1 pyrrolidine)-2 méthanol (isomère thréo)

On chauffe une nuit au reflux une solution benzénique de 4,8 g d'α-phényl pyrrolidine-2 méthanol (thréo) à laquelle on a ajouté 5 ml de triéthylamine et 3,4 g de bromo-2 éthanol. On refroidit à température ambiante, on sépare le précipite de bromhydrate de triéthylamine formé et on chasse le solvant par distillation sous pression réduite. On obtient 3,8 g d'une huile orangée qui cristallise par addition de 7,6 ml d'éther isopropylique. On obtient ainsi 3 g d'α-phényl (β-hydroxyéthyl-1 pyrrolidine)-2 méthanol (thréo), qui fond à 68° C.

Spectre de R.M.N. du produit obtenu:
- proton en α du OH: $\delta = 4{,}3$ ppm
- $CH_2OH$: $\delta = 3{,}6$ ppm

## Exemple 6

### α-(chloro-4 phényl) pyrrolidine-2 méthanol (isomère érythro)

#### a) Préparation de la (chloro-4 benzyol)-5 pyrrolidone-2

On opère comme à la lère partie de l'exemple 1 en remplaçant les 142 g d'acide pyroglutamique par 35 g de ce même acide, les 300 g de pentachlorure de phosphore par 75 g de pentachlorure de phosphore, et les trois litres de benzène anhydre par 200 ml de chlorobenzène.

On obtient 30 g de (chloro-4 benzoyl)-5 pyrrolidone-2, qui fond à 174° C.

#### b) Préparation de l'α-(chloro-4 phényl) pyrrolidine-2 méthanol (isomère érythro)

On opère comme à la deuxième partie de l'exemple 1 en remplaçant les 65,6 g de benzoyl-5 pyrrolidone-2 par 15 g de (chloro-4 benzoyl)-5 pyrrolidone-2.

On abtient 8 g d'α-(chloro-4 phényl) pyrrolidine-2 méthanol (érythro) sous forme de chlorhydrate fondant à 177° C.

Analyse élémentaire du produit obtenu:

| | | | | |
|---|---|---|---|---|
| Trouvé | C % 52,85 | H % 6,16 | N % 5,47 | Cl % 27,95 |
| Calculé | C % 53,2 | H % 6,05 | N % 5,65 | Cl % 28,6 |

## Exemple 7

### Préparation de l'α-(chloro-4 phényl) pyrrolidine-2 méthanol (isomère thréo).

On opère comme à l'exemple 2 en remplaçant les 52,4 g d'α-phényl pyrrolidine-2 méthanol (isomère érythro) par 10 g d'α-(chloro-4 phényl) pyrrolidine-2 méthanol (isomère érythro). On obtient 8 g d'α-(chloro-4 phényl) pyrrolidine-2 méthanol (thréo) sous forme de chlorhydrate fondant à 190° C.

Analyse élémentaire du produit obtenu:

| | | | |
|---|---|---|---|
| Trouvé: | C % 53,72 | H % 6,13 | N % 5,67 |
| Calculé | C % 53,23 | H % 6,05 | N % 5,65 |

## Exemple 8

### α-(cyclohexyl-4 phényl) pyrrolidine-2 méthanol (isomère érythro)

#### a) Préparation de la (cyclohexyl-4 benzoyl)-5 pyrrolidone-2

On opère comme à la lère partie de l'exemple 1 en remplaçant les 142 g d'acide pyroglutamique, les 300 g de $PCl_5$ et les trois litres de benzène anhydre par respectivement 20 g d'acide pyroglutamique, 43 g de $PCl_5$ et 25 g de cyclohexylbenzène.

On obtient 20 g de (cyclohexyl-4 benzoyl)-5 pyrrolidone-2, qui fond à 200° C.

b) Prépraration de l'α-(cyclohexyl-4 phényl) pyrrolidine-2 méthanol (isomère érythro)

On opère comme à la 2ème partie de l'exemple 1 en remplaçant les 65,6 g de benzoyl-5 pyrrolidone-2 par 10 g de (cyclohexyl-4 benzoyl)-5 pyrrolidone-2.

On obtient 7,2 g d'α-(cyclohexyl-4 phényl) pyrrolidine-2 méthanol (érythro) sous forme de chlorhydrate fondant à 210° C.

Analyse élémentaire du produit obtenu:

| | | | |
|---|---|---|---|
| Trouvé | C % 68,77 | H % 8,87 | N % 4,59 |
| Calculé | C % 69,1 | H % 8,8 | N % 4,73 |

Exemple 9

Préparation de l'α-(cyclohexyl-4 phényl) pyrrolidine-2 méthanol (isomère thréo)

On opère comme à l'exemple 2 en remplaçant les 52,4 g d'α-phényl pyrrolidine-2 méthanol (érythro) par 5 g d'α-(cyclohexyl-4 phényl) pyrrolidine-2 méthanol (érythro). On obtient 4,5 g d'α(cyclohexyl-4 phényl) pyrrolidine-2 méthanol (thréo), sous forme d'oxalate fondant à 139° C.

Analyse élémentaire du produit obtenu:

| | | | |
|---|---|---|---|
| Trouvé | C % 65,37 | H % 7,88 | N % 4,00 |
| Calculé | C % 65,33 | H % 7,74 | N % 4,01 |

Exemple 10

α-(tertiobutyl-4 phényl) pyrrolidine-2 méthanol (isomère érythro)

a) Préparation de la (tertiobutyl-4 benzoyl)-5 pyrrolidone-2

On opère comme à la lère partie de l'exemple 1 en remplaçant les 142 g d'acide pyroglutamique, les 300 g de $PCl_5$ et les 3 litres de benzène anhydre par respectivement 40 g d'acide pyroglutamique, 90 g de $PCl_5$ et 42 g de tertiobutylbenzène. On obtient 30 g de (tertiobutyl-4 benzoyl)-5 pyrrolidone-2, qui fond à 144° C.

b) Préparation de l'α-(tertiobutyl-4 phenyl) pyrrolidine-2 méthanol (isomère érythro)

On opère comme à la 2ème partie de l'exemple 1 en remplaçant les 65,6 g de benzoyl-5 pyrrolidone-2 par 15 g de (tertiobutyl-4 benzoyl)-5 pyrrolidone-2.

On obtient 10 g d'α-(tertiobutyl-4 phényl) pyrrolidone-2 méthanol (érythro) sous forme de chlorhydrate fondant à 199° C.

Analyse élémentaire du produit obtenu:

| | | | |
|---|---|---|---|
| Trouvé | C % 66,76 | H % 8,98 | N % 5,21 |
| Calculé | C % 66,8 | H % 8,9 | N % 5,19 |

Exemple 11

Préparation de l'α-(tertiobutyl-4 phényl) pyrrolidine-2 méthanol (isomère thréo)

On opère comme à l'exemple 2 en remplaçant les 52,4 g d'α-phényl pyrrolidine-2 méthanol (érythro) par 10 g d'α-(tertiobutyl-4 phényl) pyrrolidine-2 méthanol (érythro). On obtient 8 g d'α-(teriobutyl-4 phényl) pyrrolidine-2 méthanol (thréo), sous forme de chlorhydrate fondant à 190° C.

Spectre de R.M.N. du produit obtenu:

— proton en α de OH: $\delta = 4,3$ ppm

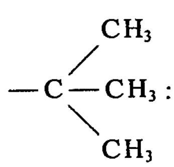 : $\delta$ = 1,4 ppm

## Proprietes Pharmacologiques

### 1°) Activité hypotriglycéridémiante

Dans le but de déterminer si les composés de la présente invention possèdent la propriété de réduire un taux anormalement élevé de triglycérides, nous avons entrepris d'induire chez l'animal une hypertriglycéridémie expérimentale. L'administration d'une quantité déterminée d'huile (huile d'olive) à l'animal 'a jeun s'est avéré à cet égard une méthode fiable, précédemment utilisée par A. BIZZ et Coll. — Europ. Journal of Pharmacology 23 (1973), 131.

Des rats mâles pesant 200 g environ, mis à jeun 18 heures auparavant, sont traités par voie orale avec les produits à étudier puis, deux heures après, on leur administre per os 20 ml/kg d'huile d'olive. Les rats sont sacrifiés deux heures après l'administration de l'huile et tués par décapitation. Le sérum est séparé par centrifugation et on dose les triglycérides sur une prise de 0,2 ml de sérum par extraction à l'aide d'un mélange heptane-isopropanol et colorimétrie suivant la méthode de VAN HANDEL et Coll. J. Lab. and Clin. Med. 1957, 50, 152—157, en utilisant un ensemble de réactifs analytiques connu sous la dénomination commerciale »Triglycerides-kit Bio Mérieux«.

Les taux de triglycérides obtenus, exprimés en mg de triglycérides (calculés en trioléine) par ml de sérum, sont comparés au taux de triglycérides des animaux témoins (animaux non traités avec les produits et auxquels on a administré 20 ml/kg per os d'huile d'olive), et on exprime les résultats par un pourcentage d'inhibition, qui représente la diminution relative en % du taux de triglycérides chez les animaux traités par rapport à ce même taux chez les animaux témoins.

Les résultats sont rassemblés dans le tableau suivant dans lequel a également été donné à titre comparatif le résultat fourni par l'acide nicotinique, produit de référence.

| Produits | Doses (mg/kg) | Voie | % d'inhibition | Signification Statistique des résultats selon le test T de Student Fischer |
|---|---|---|---|---|
| Acide nicotinique | 100 | p. o. | 50 | 0,01 |
| Exemple 1 | 50 | p. o. | 33 | 0,05 |
| Exemple 2 | 50 | p. o. | 45 | 0,01 |
| | 6,25 | p. o. | 41 | 0,01 |
| Exemple 5 | 100 | p. o. | 45 | 0,01 |
| Exemple 7 | 100 | p. o. | 41 | 0,01 |

Les produits selon l'invention sont donc de remarquables hypotriglycéridémiants.

En particulier le produit de l'exemple 2 est encore très actif à la dose de 6,25 mg/kg par voie orale.

### 2°) Action sur le système nerveux central

L'activité des produits sur le système nerveux central a été démontrée à l'aide du test d'hypothermie à la réserpine chez la souris, selon la méthode de D. M. ASKEN, Life Sciences 1963 tome 2, p. 725.

Les résultats sont exprimés par une $DE_{50}$ ou dose de produit, en mg/kg, capable d'exercer un effet antihypothermisant égal à 50% de l'effet antihypothermisant provoqué par 15 mg/kg (per os) d'imipramine.

Les resultats obtenus sont rasseblés dans le tableau suivant:

| Produit | $DE_{50}$ p. o. (mg/kg) |
|---|---|
| Exemple 1 | 4,5 |
| Exemple 2 | >150 |
| Exemple 6 | 18 |
| Exemple 7 | > 50 |

Le tableau ci-dessus met en évidence la différence d'activité sur le système nerveux central existant entre les stéréoisomères correspondant à une même formule développée. Alors que les produits de la série thréo présentent une activité très faible sur le système nerveux central, certains produits de la série érythro ont une action psychostimulante notable.

Cette différence d'activité sur le système nerveux central permet d'utiliser certains composés de formule (I) comme psychotropes et d'autres comme hypolipémiants dénués d'action sur le système nerveux central.

Proprietes Toxicologiques

Les toxicités aiguës des composés selon l'invention ont été déterminées chez la souris mâle $CD_1$ (Charles River) par voie orale. Les $DL_{50}$ ont été calculées, après trois jours d'observation, par la méthode cumulative de J. J. Reed et Coll. (Am. J. Hyg., 27 (1938) 493).

Les $DL_{50}$ obtenus sont rassemblés dans le tableau suivant:

| Produits | $DL_{50}$ (mg/kg) p. o. |
|---|---|
| Exemple 2 | 600 |
| Exemple 5 | 675 |
| Exemple 7 | 400 |
| Exemple 10 | 600 |
| Exemple 3 | 270 |
| Exemple 4 | 800 |
| Exemple 6 | 675 |
| Exemple 8 | >900 |

Les composés de formule (I) se comportent donc comme des substances relativement peu toxiques chez la souris.

Utilisation Therapeutique

Les composés de formule (I) et leurs sels avec les acides pharmaceutiquement acceptables peuvent être utilisés en thérapeutique humaine sous forme de comprimés, capsules, gélules, suppositoires, solutions ingérables ou injectables etc.... pour le traitement des dislipémies. Certains sont en outres utilisables comme psychotropes plus particulièrement comme psychostimulants.

La posologie dépend des effets recherchés et de la voie d'administration utilisée. Par exemple, par voie orale, elle peut être comprise entre 50 et 500 mg de substance active par jour, avec des doses unitaires allant de 10 à 100 mg.

**Revendications**

1. Les composés de formule:

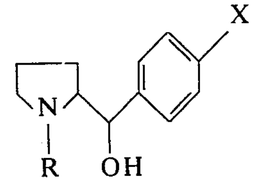

(I)

dans laquelle X est un atome d'hydrogène ou d'halogène ou un groupe alkoxy ayant 1 à 4 atomes de carbone, alkyle ou cycloalkyle ayant 1 à 8 atomes de carbone, et R représente un atome d'hydrogène, un groupe alkyle ayant 1 à 4 atomes de carbone, ou un groupe aralkyle ou hydroxyalkyle dans lequel la

chaîne alkyle a 1 à 4 atomes de carbone.

2. Procédé pour la préparation des composés de formule (I) dans lesquels R est un atome d'hydrogène, caractérisé en ce que l'on fait réagir le chlorure de l'acide pyroglutamique avec un dérivé benzénique de formule

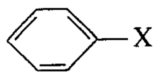

dans laquelle X a les même significations que dans la formule (I) de la revendication 1, et en ce que l'on réduit le composé de formule:

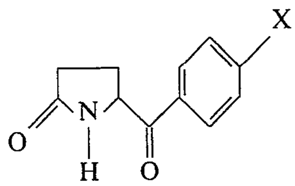

(II)

ainsi obtenu.

3. Procédé pour la préparation des composés de formule (I) pour lesquels R est autre qu'un atome d'hydrogène, caractérisé en ce qu'il consiste à soumettre à une alkylation les composés correspondants de formule (I) pour lesquels R est un atome d'hydrogène.

4. Médicament, particulièrement utile comme hypolipémiant, caractérisé en ce qu'il contient comme agent actif un composé de formule (I) ou un sel d'un tel composé avec un acide pharmaceutiquement acceptable.

5. Médicament selon la revendication 4 caractérisé en ce que le composé agent actif est l'$\alpha$-phényl pyrrolidine-2 méthanol (isomère thréo) ou un sel de ce composé avec un acide pharmaceutiquement acceptable.

**Patentansprüche**

1. Verbindung der Formel

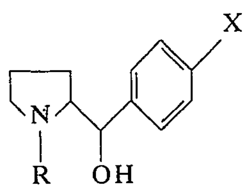

(I)

in der X ein Wasserstoff- oder Halogenatom, eine Alkoxygruppe mit 1 bis 4 Kohlenstoffatomen, eine Alkyl- oder Cycloalkylgruppe mit 1 bis 8 Kohlenstoffatomen und R ein Wasserstoffatom, eine Alkylgruppe mit 1 bis 4 Kohlenstoffatomen, oder eine Aralkyl- oder Hydroxyalkylgruppe, in der die Alkylkette 1 bis 4 Kohlenstoffatome aufweist, bedeuten.

2. Verfahren zur Herstellung der Verbindungen der Formel (I), in denen R ein Wasserstoffatom bedeutet, dadurch gekennzeichnet, daß man das Pyroglutaminsäurechlorid mit einem Benzolderivat der Formel

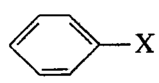

in der X die gleichen Bedeutungen wie in der Formel (I) des Anspruchs 1 hat, zur Umsetzung bringt und daß man die auf diese Weise erhaltene Verbindung der Formel

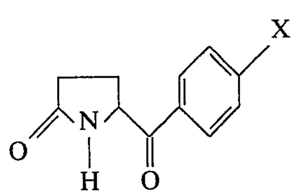

(II)

reduziert.

3. Verfahren zur Herstellung von Verbindungen der Formel (I), in denen R eine andere Bedeutung als Wasserstoff hat, dadurch gekennzeichnet, daß man die entsprechenden Verbindungen der Formel (I),

in denen R ein Wasserstoffatom bedeutet, einer Alkylierung unterwirft.

4. Pharmazeutische Zubereitung, im besonderen geeignet als Lipidsenker, dadurch gekennzeichnet, daß sie als Wirkstoff eine Verbindung der Formel (I), oder ein Salz dieser Verbindung mit einer pharmazeutisch verträglichen Säure enthält.

5. Pharmazeutische Zubereitung nach Anspruch 4, dadurch gekennzeichnet, daß der Wirkstoff α-Phenyl(pyrrolidin-2yl)-methanol (threo-Isomer) oder ein Salz dieser Verbindung mit einer pharmazeutisch verträglichen Säure ist.

## Claims

1. The compounds of the formula:

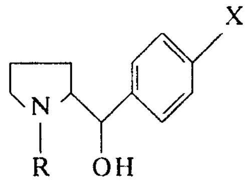

(I)

in which X is a hydrogen or halogen atom or an alkoxy group containing from 1 to 4 carbon atoms, an alkyl or cycloalkyl group containing from 1 to 8 carbon atoms, and R represents a hydrogen atom, an alkyl group containing from 1 to 4 carbon atoms or an aralkyl or hydroxyalkyl group in which the alkyl chain contains from 1 to 4 carbon atoms.

2. Process for the preparation of the compounds of the formula (I) in which R is a hydrogen atom characterised in that the chloride of pyroglutamic acid is reacted with a benzene derivative of the formula

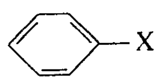

in which X has the same significance as in formula (I) of claim 1, and in that the compound thus obtained of the formula:

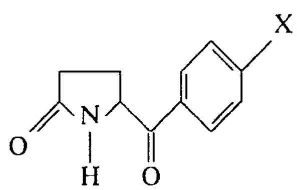

(II)

is reduced.

3. Process for the preparation of the compounds of the formula (I) in which R is other than a hydrogen atom, characterised in that it consists in subjecting to alkylation the compounds corresponding to formula (I) in which R is a hydrogen atom.

4. Medicament, especially useful as a hypolipemiant, characterised in that it contains as active agent a compound of formula (I) or a salt of such a compound with a pharmaceutically acceptable acid.

5. Medicament according to claim 4 characterised in that the active agent compound is α-phenyl-2-pyrrolidine-methanol (threo isomer) or a salt of this compound with a pharmaceutically acceptable acid.